(19) Europäisches Patentamt / European Patent Office / Office européen des brevets

(11) **EP 2 086 409 B1**

(12) **EUROPÄISCHE PATENTSCHRIFT**

(45) Veröffentlichungstag und Bekanntmachung des Hinweises auf die Patenterteilung:
**15.12.2010  Patentblatt 2010/50**

(21) Anmeldenummer: **07819256.4**

(22) Anmeldetag: **21.10.2007**

(51) Int Cl.:
**A61B 5/11** $^{(2006.01)}$    **G01L 1/24** $^{(2006.01)}$

(86) Internationale Anmeldenummer:
**PCT/EP2007/009196**

(87) Internationale Veröffentlichungsnummer:
**WO 2008/052682 (08.05.2008 Gazette 2008/19)**

(54) **VERFAHREN UND VORRICHTUNGEN ZUM MESSEN EINER TORSION EINES KÖRPERTEILS**

METHODS AND DEVICES FOR MEASURING A TORSION OF A PART OF THE BODY

PROCEDE ET DISPOSITIFS DE MESURE D'UNE TORSION D'UNE PARTIE D'UN CORPS

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **02.11.2006  DE 102006051742**

(43) Veröffentlichungstag der Anmeldung:
**12.08.2009  Patentblatt 2009/33**

(60) Teilanmeldung:
**10007464.0 / 2 255 727**

(73) Patentinhaber: **SIEMENS AKTIENGESELLSCHAFT**
**80333 München (DE)**

(72) Erfinder:
• **GOLDBECK, Dirk, David**
  **81927 München (DE)**
• **HAPPEL, Tobias**
  **14089 Berlin (DE)**
• **L'HENORET, Benjamin**
  **31000 Toulouse (FR)**

(56) Entgegenhaltungen:
WO-A-00/35345    WO-A-89/11247
US-A- 6 127 672

**Beschreibung**

Die vorliegende Erfindung beschäftigt sich mit Verfahren, Vorrichtungen und einer Verwendung einer jeweiligen Vorrichtung zum Messen einer Torsion eines Körpers mit Hilfe von Biegesensoren.

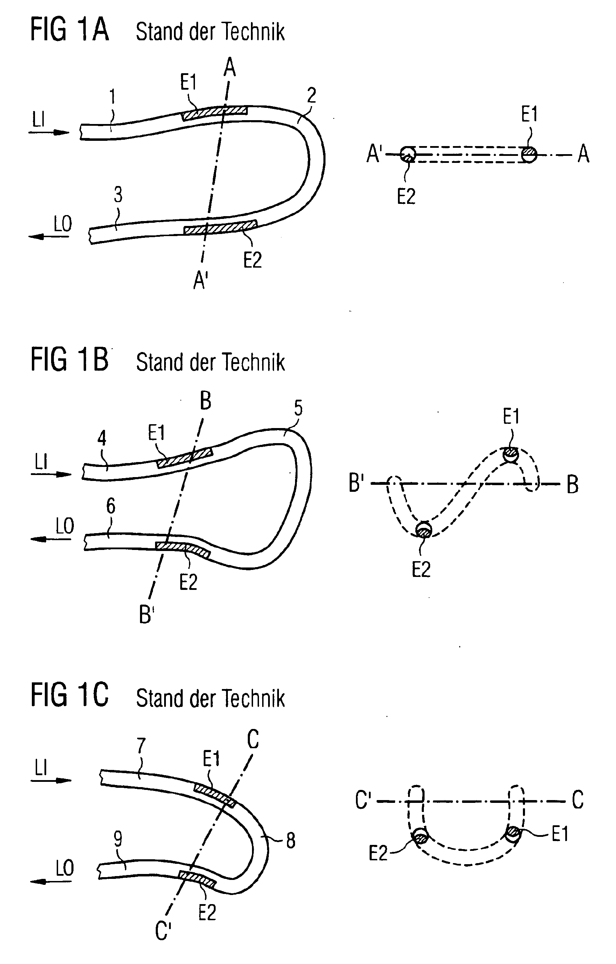

Aus einem US-Patent [1] ist bekannt, dass ein faseroptischer Biegesensor zum Messen einer Biegung eines Fingers eingesetzt werden kann. Ferner ist in einem weiteren US-Patent [2] neben verschiedenen faseroptischen Biegesensoren auch ein faseroptischer Sensor gemäß den Figuren 18-22 angegeben, mit dem eine Torsion messbar ist. Hierbei ist der Sensor zur Messung der Torsion derart ausgebildet, dass eine Transmissionsänderung bei einer Torsion messbar ist. Der in [2] angegebene Sensor zeigt jedoch den Nachteil, dass bei einer Flexion, d.h. Beugung, eine Torsion angezeigt wird. Zudem wird bei einer gleichzeitigen Flexion und Torsion die Torsion fehlerhaft gemessen. Mit Hilfe der Figuren 1A-C wird dieser Nachteil näher erläutert.

In Figur 1A ist eine Schrägansicht einer Schlaufe eines faseroptischen Biegesensors zu sehen, bei dem eintretendes Licht LI in die Faser eingekoppelt und nach dem Durchgang durch die Schlaufe austretendes Licht LO aus der Faser austritt. Das austretende Licht LO wird bspw. durch eine Photodiode oder lichtempfindlichen Sensor verarbeitet. Das Verhältnis aus aus- und eintretendem Licht LO/LI gibt eine Dämpfung durch die gesamte Faserschlaufe wieder. In der Faser befinden sich zwei Bereiche E1, E2 mit jeweiligen Einkerbungen, wobei in Abhängigkeit vom Radius einer Biegung im Bereich der jeweiligen Einkerbung die jeweilige Transmission des eintretenden Lichts variiert wird. Einer der Bereiche ist auf der Oberseite und der andere Bereich auf der Unterseite der Faser angebracht. Im Querschnitt A'---A ist die Lage der Bereiche E1, E2 erkennbar. Werden LI = 100% Licht an einer Stelle (1) eingekoppelt, so werden bspw. 80% davon durch den Bereich E1 gedämpft, d.h. an der Stelle (2) ist die Lichtleistung 80% von LI. Durch den Bereich E2 werden auch 80% der in diesen Bereich eintretenden Lichtleistung gedämpft, so dass an der Stelle (3) für das austretende Licht gilt:

$$LO = 80\% * 80\% * LI = 0,64 * LI$$

Figur 1A entspricht der Situation aus den Figuren 18-22 des Dokuments [2]. In Figur 1A ist der Torsionssensor in seiner Grundstellung.

Bei einer Torsion verdrehen sich die Bereiche E1, E2 gegenüber den Orten, an denen das Licht ein bzw. ausgekoppelt wird. Dies ist in dem Querschnitt B'---B zu sehen. Hierbei nimmt bei beiden Bereichen E1, E2 die Dämpfung, bzw. die Transmission, des Lichts gleichermaßen zu oder ab, je nach Richtung der Torsion. Werden an der Stelle (4) LI = 100% Licht eingekoppelt so sind es an der Stelle (5) noch 90% und an der Stelle (6) 90%*90%, so dass sich für das austretende Licht LO ergibt:

$$LO = 90\% * 90\% * LI = 0,81 * LI$$

Ist die Torsion in einer zu diesem Beispiel entgegen gesetzten Richtung, das heißt die Dämpfung nimmt in den Bereichen E1, E2 gegenüber der Grundstellung zu, so ergibt sich das austretende Licht LO bzw. zu:

$$LO = 60\% * 60\% * LI = 0,36 * LI$$

Da die Faser elastisch ist kann davon ausgegangen werden, dass sich die bei einer Torsion einstellenden Biegeradien an den Bereichen E1, E2 nahezu identisch sind, sodass auch die Dämpfung zw. Transmission des Lichts nahezu identisch ist.

Schließlich wird in Figur 1C der Fall betrachtet, dass die Schlaufe keiner Torsion sondern einer Flexion, d.h. Biegung, ausgesetzt wird. In Figur 1C ist die Schlaufe nach unten gebogen. Dies zeigt sich auch in dem dazugehörigen Querschnitt C'---C. Bei wird die Dämpfung im Bereich E1 größer und im Bereich E2 kleiner als in der Grundstellung gemäß Figur 1A. Wird das an der Stelle (7) zu LI = 100% eintretende Licht durch den Bereich E1 um 45% gedämpft, d.h. an der Stelle (8) ist die Lichtleistung noch 55%, so kann die Lichtleistung an der Stelle (9) nur noch 55% oder weniger betragen, da der Bereich E2 auch eine Dämpfung des Lichts durchführt, d.h. keine Kompensation des durch den Bereich E1 gedämpften Lichts durchführen kann. In diesem Beispiel ist LO = 0,45 * LI, wodurch eine Torsion erkannt wird, da LO/LI kleiner als die Grundstellung, das heißt 64%, ist.

Somit zeigt sich, dass der in [2] vorgeschlagene Torsionssensor bei einer Flexion des Sensors auch Torsion misst, obwohl keine Torsion vorhanden ist. Der gleiche Nachteil ergibt sich ferner, wenn Torsion und Flexion, d.h. Beugung, gleichzeitig auftreten, wobei in diesem Fall die Torsion aufgrund des Einflusses der Flexion auf das Messergebnis fehlerhaft ermittelt wird.

In einem Dokument WO 00/35345 wird eine Methode und eine Vorrichtung zum Überwachen von Haltungsfehlern der Wirbelsäule vorgestellt.

Somit stellt sich die zu lösende Aufgabe derart, ein Verfahren und eine Vorrichtung anzugeben, die eine Torsion mit Hilfe von Biegesensoren auch bei Vorhandensein von Flexion zuverlässig ermitteln.

Diese Aufgabe wird durch die unabhängigen Ansprüche gelöst. Weiterbildungen der Erfindung sind den abhängigen Ansprüchen zu entnehmen.

Die Erfindung betrifft ein Verfahren zum Messen einer Torsion eines Körpers mit Hilfe von Biegesensoren, wobei die Biegesensoren auf einer Oberfläche des Körpers angebracht sind,
mit folgenden Schritten:

> Messen einer jeweiligen durch die Torsion auftretenden Biegung der Biegesensoren mittels eines ersten Biegesignal des ersten Biegesensors und eines zweiten Biegesignals des zweiten Biegesensors;
> Ermitteln eines für die Torsion charakteristischen Biegesignals durch Subtraktion des ersten Biegesignals von dem zweiten Biegesignal.

Je nach Torsionsrichtung ist die Verbiegung bspw. links- und rechtsseitig einer Torsionsachse des Körpers angebrachter Biegesensoren unterschiedlich. Die zwei Biegesensoren werden deshalb unterschiedlich stark ausgeprägte Biegesignale, das heißt Transmissionswerte bei faseroptischen Biegesensoren, liefern. Durch die Verknüpfung des ersten Biegesignals mit dem zweiten Biegesignal durch Subtraktion wird erreicht, dass lediglich die Torsionsbewegung gemessen wird, da ein Anteil einer Flexionsbewegung durch die Subtraktion der beiden Biegesignale eliminiert wird. Beispielsweise kann als Biegesensor ein faseroptischer Biegesensor eingesetzt werden, wobei der erste Biegesensor links und der zweite Biegesensor rechts der Wirbelsäule auf dem Rücken angebracht werden kann. Die Biegesensoren liefern je nach Biegerichtung unterschiedliche Biegesignale. So wird bspw. bei einer Biegung des faseroptischen Biegesensors in eine Richtung eine für die Biegung charakteristische Lichtdämpfung zunehmen, wohingegen eine Biegung in die andere Richtung eine Abnahme der Lichtdämpfung bewirkt.

Vorzugsweise bewirkt die auftretende Biegung des ersten und zweiten Biegesignals in einer selben Raumrichtung bei sowohl dem ersten als auch dem zweiten Biegesignal eine Zunahme oder Abnahme. Hierdurch wird erreicht, dass die Torsion mittels des charakteristischen Biegesignals in einer zuverlässigen Weise messbar ist.

In einer vorzugsweisen Weiterbildung werden vor einer Durchführung der Torsion ein erstes Basisbiegesignal des ersten Biegesensors und ein zweites Basisbiegesignal des zweiten Biegesensors gemessen, ein charakteristisches Basisbiegesignal durch Subtraktion des ersten und des zweiten Basisbiegesignals erstellt und nach einer Durchführung der Torsion das charakteristische Basisbiegesignal von dem ermittelten charakteristischen Biegesignal subtrahiert.

Mit dieser Weiterbildung wird erreicht, dass im Ruhezustand, d.h. vor der Torsion, des Körpers das charakteristische Biegesignal Null ist, und somit Messungenauigkeiten der Torsion durch fehlende Kalibrierung des charakteristischen Biegesignals im Ruhezustand vermieden wird.

Ferner ist Teil der Erfindung auch ein alternatives Verfahren zum Messen einer Torsion eines Körpers mit Hilfe von Biegesensoren, wobei die Biegesensoren auf einer Oberfläche des Körpers angeordnet sind, mit folgenden Schritten:

> Messen einer jeweiligen durch die Torsion auftretenden Biegung an den Biegesensoren mittels eines ersten Biegesignals des ersten Biegesensors und mittels eines zweiten Biegesignals des zweiten Biegesensors, wobei die Biegung des ersten und zweiten Biegesensors in eine selbe Raumrichtung eine Zunahme des ersten Biegesignals und eine Abnahme des zweiten Biegesignals bewirkt;
> Ermitteln eines für die Torsion charakteristischen Biegesignals durch Addition des ersten Biegesignals und des zweiten Biegesignals.

Hierbei sind die gleichen Vorteile wie bei dem erläuterten Verfahren erzielbar, wobei bei dem alternativen Verfahren lediglich die Generierung des für die Torsion charakteristischen Biegesignals anstelle durch Subtraktion mit einer Addition bewerkstelligt wird.

Vorzugsweise werden bei dem alternativen Verfahren vor einer Durchführung der Torsion ein erstes Basisbiegesignal des ersten Biegesensors und ein zweites Basisbiegesignal des zweiten Biegesensors gemessen, ein charakteristisches Basisbiegesignal durch Addition des ersten und des zweiten Basisbiegesignals erstellt und nach einer Durchführung der Torsion das charakteristische Basisbiegesignal von dem ermittelten charakteristischen Biegesignal subtrahiert. Hierbei sind die erzielbaren Vorteile anlog zum dem obigen Verfahren.

In einer vorzugsweisen Weiterbildung beider Verfahren können Biegesensoren eingesetzt werden, die auf der Oberfläche des Körpers nahezu parallel zur Achse der Torsion angebracht sind. Hiermit können die die Torsion repräsentativen Biegesignale und somit die Torsion selbst in einer guten Genauigkeit gemessen werden. Unter nahezu parallel ist im Rahmen dieser Erfindung zu verstehen, dass auch kleine Abweichungen von der parallelen Anordnung als parallel verstanden werden soll, wie beispielsweise $\pm 1$ cm oder kleiner.

Werden vorzugsweise die Biegesensoren eingesetzt, die eine jeweilige sensitive Zone mit einer jeweiligen örtlichen Raumausdehnung in Richtung der Achse aufweisen, so können punktuelle Ungenauigkeiten in der Messung der jeweiligen Biegung vermieden werden, da die Biegesignale die jeweilige Biegung über einen örtlichen Bereich des Körpers repräsentieren. Ferner bewirkt die Anordnung in der jeweiligen örtlichen Raumausdehnung in Richtung der Achse, dass bereits kleine Torsionsbewegungen durch die Biegesensoren messbar sind.

Vorzugsweise wird ein zu der Torsion gehörender Torsionswinkel mittels des charakteristischen Biegesignals auf Basis einer Umrechnungsfunktion erstellt. Hierdurch kann die Torsion in einfacher Weise durch den Torsionswinkel beschrieben werden. Die Umrechungsfunktion gibt hierbei eine Relation des charakteristischen Biegesignals zu dem dazugehörigen Torsionswinkel an. Diese Relation ist bspw. linear oder gleicht Nichtlinearitäten der Biegesensoren, z.B. kurz vor der maximalen Biegung der Biegesensoren, aus.

Vorzugsweise werden Paare erster und zweiter Biegesignale über der Zeit gemessen und ein Zeitverlauf der Torsion durch Generieren des jeweiligen charakteristischen Biegesignals jeweiliger Paare des ersten und zweiten Biegesignals ermittelt. Hiermit kann in einfacher Weise eine Aussage über die Torsionsbewegung über der Zeit dargestellt werden. Dies ist beispielsweise wichtig, um ungünstige Bewegungsabläufe, beispielsweise des Rückens eines Patienten, ermitteln zu können.

Zudem kann für die beiden genannten Verfahren vor der Ermittlung des charakteristischen Biegesignals eine Gewichtung der jeweiligen Biegesignale vorgenommen werden, um bspw. ungleiche Messergebnisse der Biegesensoren bei identischer Biegung auszugleichen.

In einer vorzugsweisen Weiterbildung der Erfindung sind der Körper als Rücken eines Patienten und die Achse als Wirbelsäule des Patienten ausgebildet. Gerade bei der Vermessung der Torsion der Wirbelsäule lassen sind ein oder mehrere der vorangegangen Verfahrenschritte gut anwenden.

Vorzugsweise werden Biegesensoren eingesetzt, die als faseroptische Biegesensoren ausgebildet sind. Diese zeigen den Vorteil, dass Sie weder gegen elektromagnetische Strahlung anfällig sind, noch selbst diese Strahlung emittieren. Zudem haben faseroptische Biegesensoren ein geringes Gewicht und sind preiswert herstellbar, so dass diese zur Vermessung der Wirbelsäule in einem Massenmarkt gut einsetzbar sind.

Die Erfindung betrifft auch eine Vorrichtung zum Messen einer Torsion eines Körpers, mit folgenden Mitteln:

erster Biegesensor und zweiter Biegesensor, die an einer Oberfläche des Körpers angeordnet sind;
Messmittel zum Messen der jeweiligen durch die Torsion auftretenden Biegung an den Biegesensoren mittels eines ersten Biegesignals des ersten Biegesensors und mittels eines zweiten Biegesignals des zweiten Biegesensors;
Auswertemittel zum Ermitteln eines für die Torsion charakteristischen Biegesignals durch Subtraktion des ersten Biegesignals von dem zweiten Biegesignal.

Vorzugsweise sind der erste und der zweite Biegesensoren derart angeordnet, dass die auftretende Biegung des ersten und zweiten Biegesensors in eine selbe Raumrichtung eine Zunahme oder Abnahme des ersten und des zweiten Biegesignals bewirkt.

In einer Weiterbildung der Vorrichtung kann das Messmittel vor einer Durchführung der Torsion ein erstes Basisbiegesignal des ersten Biegesensors und ein zweites Basisbiegesignal des zweiten Biegesensors messen und das Auswertemittel zum ein charakteristischen Basisbiegesignals durch Subtraktion des ersten und des zweiten Basisbiegesignals erstellen und das charakteristischen Basisbiegesignals von dem ermittelten charakteristischen Biegesignal subtrahieren.

Durch die voranstehenden Vorrichtungsmerkmale sind dieselben Vorteile wie durch die dazu korrespondierenden Verfahrensmerkmale erzielbar.

Die Vorrichtung kann sich weiterhin dadurch auszeichnen, dass der erste und zweite Biegesensor jeweils als faseroptischer Biegesensor mit einer jeweiligen sensitiven Zone ausgebildet ist, wobei die sensitiven Zonen derart in einem Kern-Mantelübergang des jeweiligen Biegesensors ausgebildet sind, dass die sensitiven Zonen in eine selben Raumrichtung zeigen, insbesondere senkrecht in den Körper oder senkrecht aus dem Körper. Durch diese spezifische Anordnung der sensitiven Zonen der Biegesensoren wird eine hohe Empfindlichkeit bei einer Messung der durch die Torsion sich ergebenden Biegungen der Biegesensoren erreicht.

Zudem ist Teil der Erfindung eine alternative Vorrichtung zum Messen einer Torsion eines Körpers, mit folgenden Mitteln:

Ein erster Biegesensor und ein zweiter Biegesensor, die auf einer Oberfläche des Körpers angeordnet sind, wobei die Biegung des ersten und zweiten Biegesensors in eine selbe Raumrichtung eine Zunahme eines ersten Biegesignals und eine Abnahme eines zweiten Biegesignals bewirkt;

Ein Messmittel zum Messen der jeweiligen durch die Torsion auftretenden Biegung an den Biegesensoren mittels eines ersten Biegesignals des ersten Biegesensors und mittels eines zweiten Biegesignals des zweiten Biegesensors; Auswertemittel zum Ermitteln eines für die Torsion charakteristischen Biegesignals durch Addition des ersten Biegesignals und des zweiten Biegesignals.

Diese alternative Vorrichtung zeichnet sich vorzugsweise dadurch aus, dass das Messmittel vor einer Durchführung der Torsion ein erstes Basisbiegesignal des ersten Biegesensors und ein zweites Basisbiegesignal des zweiten Biegesensors misst und das Auswertemittel zum Erstellen eines charakteristischen Basisbiegesignals durch Addieren des ersten und des zweiten Basisbiegesignals und zum Subtrahieren des charakteristischen Basisbiegesignals von dem ermittelten charakteristischen Biegesignal ausgestaltet ist.

Vorzugsweisen sind die Biegesensoren an der Oberfläche des Körpers nahezu parallel zur Achse der Torsion angebracht.

In einer Weiterbildung können die Biegesensoren eine jeweilige sensitive Zone aufweisen, wobei die sensitiven Zonen mit einer jeweiligen örtlichen Raumausdehnung in Richtung der Achse angeordnet sind.

In einer vorzugsweisen Weiterbildung kann das Auswertemittel ein zu der Torsion gehörenden Torsionswinkel mittels des charakteristischen Biegesignals auf Basis einer Umrechnungsfunktion erstellen.

Vorzugsweise kann das Messmittel ein jeweiliges Paar erster und zweiter Biegesignale über der Zeit messen und das Auswertemittel einen Zeitverlauf der Torsion durch Generieren des jeweiligen charakteristischen Biegesignals jeweiliger Paare des ersten und zweiten Biegesignals ermitteln.

In einer optionalen Weiterbildung der jeweiligen Vorrichtung sind der Körper als Rücken eines Patienten und die Achse als Wirbelsäule des Patienten ausgebildet.

Vorzugsweise sind Biegesensoren in der jeweiligen Vorrichtung eingesetzt, die als faseroptische Biegesensoren ausgebildet sind.

Durch die voranstehenden Vorrichtungsmerkmale der Vorrichtung bzw. alternativen Vorrichtung sind dieselben Vorteile wie durch die dazu korrespondierenden Verfahrensmerkmale erzielbar.

Schließlich umfasst die Erfindung eine Verwendung der Vorrichtung bzw. die alternative Vorrichtung mit zumindest einem der voranstehenden Vorrichtungsmerkmale, wobei die Vorrichtung bzw. alternative Vorrichtung zur Messung der Torsion eines menschlichen Körperteils, insbesondere des Rückens mit der Wirbelsäule als Achse der Torsion, verwendet wird.

Die Verfahren bzw. die Vorrichtungen lassen sich in besonders effizienter Weise zur Vermessung der Torsion der Wirbelsäule einsetzen, da die jeweilige Vorrichtung kostengünstig für einen Massenmarkt herstellbar, auf dem Rücken des Patienten gut tragbar und keine für den Patienten gefährliche Strahlung abgibt.

Die Erfindung und Ihre Weiterbildungen werden anhand von Figuren näher erläutert. Im Einzelnen zeigen die Figuren:

Figur 1     ein aus einem Stand der Technik bekanntem Torsions- sensor

Figur 2     eine Kennlinie eines faseroptischen Biegesensors mit einer Transmissionsänderung über einem Biegera- dius

Figur 3     ein Patient, bei dem mit Hilfe von zwei Biegesenso- ren eine Torsion der Wirbelsäule ermittelt werden soll

Figur 4     ein zeitlicher Verlauf jeweiliger Transmissionswer- te der beiden Biegesensoren

Figur 5     eine beispielhafte Zuordnung eines Torsionswinkels zu einem für die Torsion charakteristischen Trans- missionswert

Figur 6     Vorrichtung zum Durchführen des Verfahrens

Elemente mit gleicher Funktion und Wirkungsweise sind in den Figuren mit denselben Bezugszeichen versehen.

Die nachfolgenden Ausführungsbeispiele werden anhand von faseroptischen Biegesensoren zur Messung einer Torsion einer Wirbelsäule näher erläutert. Die Erfindung ist jedoch nicht hierauf beschränkt. Vielmehr kann jede Art von Biegesensoren, z.B. piezo-elektrischer Biegesensor, zur Messung eines beliebigen Körpers, z.B. eine Glasplatte oder ein Holzwürfel, eingesetzt werden. In den nachfolgenden Ausführungsbeispielen zum Messung der Torsion mit faseroptischen Biegesensoren wird deshalb als erstes bzw. zweites Biegesignal ein Begriff erster bzw. zweiter Transmissionswert und als charakteristisches Biegesignal das Wort charakteristischer Transmissionswert verwendet, ohne dass die Erfindung auf diese konkrete Ausführung geschränkt sein soll.

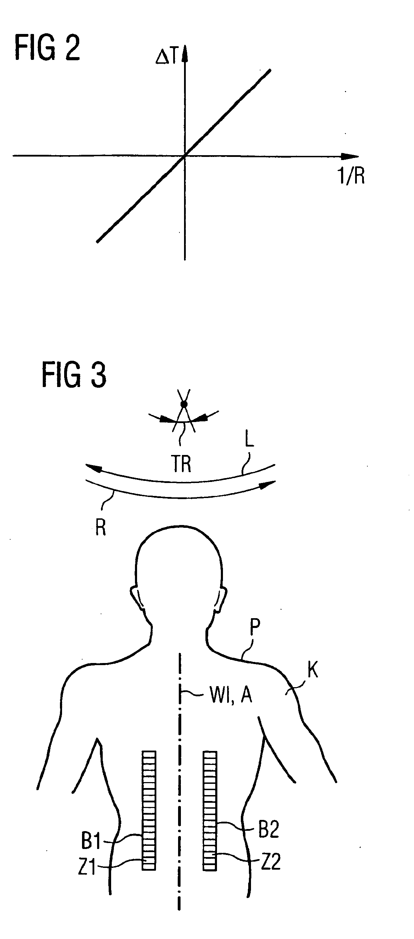

Faseroptische Biegensensoren sind bspw. aus [1] bekannt. Dabei werden über einen Bereich der Faser Einkerbungen in die Faser eingebracht, wobei bei Veränderung eines Biegeradius der Faser in diesem Bereich eine Transmission von durch die Faser tretendes Licht in Abhängigkeit des Biegeradius verändert wird. Ist der Biegeradius R unendlich, das heißt die Faser weist keine Biegung in dem Bereich auf und somit 1/R = 0, so stellt sich eine Grundtransmission ein, siehe Figur 2 im Ursprung. Wird die Faser in eine Richtung gebogen, so wird mehr Licht ausgekoppelt als im ungebogenen Zustand der Faser, so dass die Transmission gegenüber der Grundtransmission abnimmt, siehe in Figur 2 auf der linke Abschnitt der Abszisse 1/R von dem Nullpunkt. Das bedeutet, dass eine Differenztransmission $\Delta T$ kleiner Null ist, $\Delta T < 0$. Wird die Faser in die andere Richtung gebogen, so werden die Einkerbungen näher zusammengebracht, so dass weniger Licht in dem Bereich ausgekoppelt und somit die Transmission gegenüber der Grundtransmission erhöht wird, siehe in Figur 2 auf der rechten Seite der Abszisse 1/R vom Nullpunkt. Das bedeutet, dass eine Differenttransmission $\Delta T$ größer Null ist, $\Delta T > 0$. Im Folgenden wird als Biegesensor ein faseroptischer Biegesensor gemäß der obigen Erläuterung verstanden. Die Transmission ist damit in Abhängig von dem Radius R der Biegung des Bereichs darstellbar.

Zur Messung der Torsion eines Rückens, d.h. eines Körpers K, eines Patienten P werden jeweils links und rechts von der Wirbelsäule WI, die eine Achse A der Torsion darstellt, ein erster Biegesensor B1 und ein zweiter Biegesensor B2 befestigt. Im Ausführungsbeispiel gemäß Figur 3 werden die Biegesensoren jeweils nahezu parallel der Wirbelsäule angebracht. Unter nahezu parallel ist im Rahmen dieser Erfindung zu verstehen, dass auch kleine Abweichungen von der parallelen Anordnung als parallel verstanden werden soll, wie beispielsweise $\pm 1$ cm. Gemäß diesen Ausführungen sind die Einkerbungen, d.h. sensitive Zonen Z1, Z2, beider Biegesensoren bezogen auf die Ebene des Rückens entweder zeigend in Richtung des Rückens oder in die vom Rücken wegzeigende Richtung, d.h. in dieselbe Raumrichtung. In Figur 3 sind die Einkerbungen in der vom Rücken weg zeigenden Richtung angeordnet.

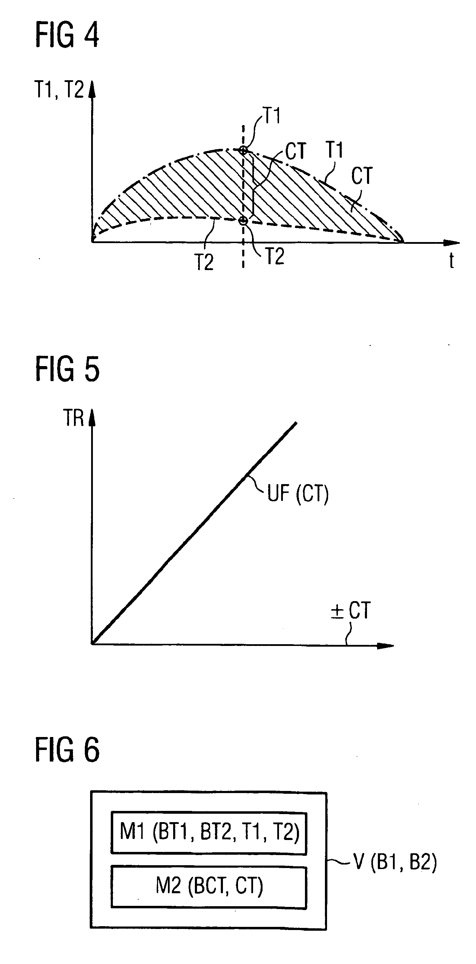

Nachdem der Patient eine Torsionsbewegung seines Rückens und somit seiner Wirbelsäule durchgeführt hat, wird eine jeweilige Transmission in Form eines ersten und zweiten Transmissionswerts T1, T2 des ersten und des zweiten Biegesensors B1, B2 ermittelt. Wird diese Ermittlung über der Zeit t laufend durchgeführt, so ergibt sich ein Transmissionsprofil des ersten und des zweiten Transmissionswertes T1, T2 beispielsweise gemäß Figur 4.

Zur Ermittlung der Torsion wird aus dem ersten und dem zweiten Transmissionswert T1, T2 ein für die Torsion charakteristischer Transmissionswert CT durch folgende Gleichung (1) ermittelt:

$$CT = T1(B1) - T2(B2) \qquad (1)$$

Der Betrag des charakteristischen Transmissionswertes CT ist ein Maß für die Stärke der Torsion und ein Vorzeichen des charakteristischen Transmissionswertes CT gibt die Richtung der Torsion an. Ist der erste Biegesensor B1 links und der zweite Biegesensor B2 rechts der Wirbelsäule, d.h. der Achse A der Torsion angebracht, so zeigt folgende Tabelle die Richtung der Torsion an, wobei die Richtung in Figur 2 mit entsprechenden Pfeilen L bzw. R eingetragen ist:

| Vorzeichen des charakteristischer Transmissionswertes CT | Richtung der Torsion |
|---|---|
| CT > 0 | Links L |
| CT < 0 | Rechts R |

In einer optionalen Erweiterung wird vor der Durchführung der Messung der Torsion eine Kalibrierung es charakteristischen Transmissionswertes CT durchgeführt, damit dieser bei einer Stellung des Körperteils ohne Torsion einen Wert 0 anzeigt. Hierzu kann eine Messung eines ersten und zweiten BasisBiegesignals bzw. -Transmissionswertes BT1, BT2 vor der Torsion erfolgen, aus denen zunächst gemäß Gleichung (1) ein Kalibrierungswert, d.h. ein charakteristisches Basisbiegesignal BCT, ermittelt wird, d.h.

$$BCT = BT1(B1) - BT2(B2) \quad (2).$$

Bei der Messung der Torsion nach einer Torsionsbewegung wird dann das charakteristisches Basisbiegesignal BCT von der Differenz des zweiten von dem ersten Transmissionswerts T2, T1 abgezogen, um bei im Ruhezustand des Rückens von einer Torsion einen Wert Null zu erhalten. Dies kann durch folgende Gleichung (3) beschrieben werden:

$$CT = T1(B1) - T2)(B2) - BCT \quad (3)$$

Ferner kann dem charakteristischen Transmissionswert CT ein Torsionswinkel TR zugeordnet werden, wobei durch den Betrag des charakteristischen Transmissionswertes CT der Betrag des Torsionswinkels TR und durch das Vorzeichen des charakteristischen Transmissionswertes CT das Vorzeichen bzw. eine Richtung des Torsionswinkels TR bestimmt werden kann. Figur 5 zeigt exemplarisch eine Umrechungsfunktion UF, mit der der Betrag des charakteristischen Transmissionswertes CT in den Betrag des Torsionswinkels TR überführt werden kann. Diese Umrechnungsfunktion muss an die spezifischen Eigenheiten der verwendeten Biegesensoren angepasst werden. Im Beispiel gemäß Figur 5 sind der Betrag des charakteristischen Transmissionswertes CT und der Betrag des Torsionswinkels TR linear voneinander abhängig. Im Allgemeinen kann mit Hilfe der Umrechungsfunktion eine Linearisierung des charakteristischen Transmissionswertes CT in den Torsionswinkel stattfinden. In diesem Beispiel ist das Vorzeichen des Torsionswinkels identisch zu dem Vorzeichen des charakteristischen Transmissionswertes CT.

Neben der Berechnung der Torsion bzw. des Torsionswinkels für ein Paar an ersten und zweiten Transmissionswerten kann die Torsion und der Torsionswinkel auch über der Zeit ermittelt und ausgegeben werden, bspw. auf einem Display. Hierbei wird für jedes Paar der über der Zeit gemessenen ersten und zweiten Transmissionswerte die Torsion bzw. der Torsionswinkel ermittelt und in Form einer Grafik oder Tabelle über der Zeit dargestellt.

Figur 6 zeigt eine Vorrichtung zum Messen einer Torsion eines Körperteils mit Hilfe von Biegesensoren, wobei die Biegesensoren als faseroptische Biegesensoren ausgebildet sind, mit einem Messmittel zum Messen eines ersten Transmissionswertes des ersten Biegesensors und eines zweiten Transmissionswertes des zweiten Biegesensors nach einer Torsionsbewegung und mit einem Auswertemittel zum Ermitteln eines für die Torsion charakteristischen Transmissionswertes durch Subtraktion des ersten Transmissionswertes von dem zweiten Transmissionswert. Ferner kann das Messmittel und/oder das Auswertemittel ferner ausgebildet sind, das Verfahren gemäß zumindest einer Erweiterung auszuführen. Das Messmittel und/oder das Auswertemittel können in Software, Hardware und/oder in einer Kombination aus Software und Hardware ausgebildet sein.

Die Erfindung wurde anhand von Ausführungsbeispielen näher erläutert. Neben den gezeigten Ausführungsformen sind im Rahmen der Erfindung auch Variationen zu verstehen. Beispielsweise kann das charakteristische Biegesignal durch Subtraktion des ersten Biegesignals von dem zweiten Biegesignal ermittelt werden. Ferner kann die jeweilige sensitiven Zone der Biegesensoren, d.h. diejenigen Raumrichtung, bei der eine Biegung durch die Biegesensoren messbar ist, in entgegengesetzten Raumrichtungen orientiert sein, sodass das charakteristische Biegesignal durch Addition der ersten und zweiten Biegesignale ermittelt werden kann. Die in den Beispielen aufgezeigten Varianten können auch in Kombination verwendet werden.

Literaturangabe

[1] US 5,097,252
[2] US 6, 127, 672

**Patentansprüche**

1.   Verfahren zum Messen einer Torsion eines Körpers (K) mit Hilfe von Biegesensoren (B1, B2), wobei die Biegesensoren auf einer Oberfläche des Körpers (K) angeordnet sind,
mit folgenden Schritten:

Messen einer jeweiligen durch die Torsion auftretenden Biegung an den Biegesensoren (B1, B2) mittels eines ersten Biegesignals (T1) des ersten Biegesensors (B1) und mittels eines zweiten Biegesignals (T2) des zweiten Biegesensors (B2), wobei die Biegung des ersten und zweiten Biegesensors (B1, B2) in eine selbe Raumrichtung eine Zunahme des ersten Biegesignals (T1) und eine Abnahme des zweiten Biegesignals (T2) bewirkt;
Ermitteln eines für die Torsion charakteristischen Biegesignals (CT) durch Addition des ersten Biegesignals (T1) und des zweiten Biegesignals (T2).

2.   Verfahren nach Anspruch 1,

**dadurch gekennzeichnet, dass**

vor einer Durchführung der Torsion ein erstes Basisbiegesignal (BT1) des ersten Biegesensors (B1) und ein zweites Basisbiegesignal (BT2) des zweiten Biegesensors (B1) gemessen wird,

ein charakteristisches Basisbiegesignal (BCT) durch Addition des ersten und des zweiten Basisbiegesignals (BT1, BT2) erstellt wird,

nach einer Durchführung der Torsion das charakteristische Basisbiegesignal (BCT) von dem ermittelten charakteristischen Biegesignal (CT) subtrahiert wird.

3. Verfahren nach einem der vorangehenden Ansprüche,
   **dadurch gekennzeichnet, dass**
   Biegesensoren (B1, B2) eingesetzt werden, die auf der Oberfläche des Körpers (K) nahezu parallel zur Achse (A) der Torsion angebracht sind.

4. Verfahren nach einem der vorangehenden Ansprüche,
   **dadurch gekennzeichnet, dass**
   Biegesensoren (B1, B2) eingesetzt werden, die eine jeweilige sensitive Zone (Z1, Z2) mit einer jeweiligen örtlichen Raumausdehnung in Richtung der Achse (A) aufweisen.

5. Verfahren nach einem der vorangehenden Ansprüche,
   **dadurch gekennzeichnet, dass**
   ein zu der Torsion gehörender Torsionswinkel (TR) mittels des charakteristischen Biegesignals (CT) auf Basis einer Umrechnungsfunktion (UF) erstellt wird.

6. Verfahren nach einem der vorangehenden Ansprüche,
   **dadurch gekennzeichnet, dass**
   ein jeweiliges Paar erster und zweiter Biegesignale (T1, T2) über der Zeit (t) gemessen werden,
   ein Zeitverlauf der Torsion durch Generieren des jeweiligen charakteristischen Biegesignals (CT) jeweiliger Paare des ersten und zweiten Biegesignals (T1, T2) ermittelt wird.

7. Verfahren nach einem der vorangehenden Ansprüche,
   **dadurch gekennzeichnet, dass**
   der Körper (K) als Rücken eines Patienten und die Achse (A) als Wirbelsäule des Patienten ausgebildet sind.

8. Verfahren nach einem der vorangehenden Ansprüche,
   **dadurch gekennzeichnet, dass**
   Biegesensoren eingesetzt werden, die als faseroptische Biegesensoren ausgebildet sind.

9. Verfahren nach einem der vorangehenden Ansprüche,
   **dadurch gekennzeichnet, dass**
   vor der Ermittlung des charakteristischen Biegesignals eine Gewichtung der jeweiligen Biegesignale durchgeführt werden.

10. Vorrichtung (V) zum Messen einer Torsion eines Körpers (K),
    mit folgenden Mitteln:

    erster Biegesensor (B1) und zweiter Biegesensor (B2), die auf einer Oberfläche des Körpers (K) angeordnet sind, wobei die Biegung des ersten und zweiten Biegesensors (B1, B2) in eine selbe Raumrichtung eine Zunahme eines ersten Biegesignals (T1) und eine Abnahme eines zweiten Biegesignals (T2) bewirkt;
    Messmittel (M1) zum Messen der jeweiligen durch die Torsion auftretenden Biegung an den Biegesensoren (B1, B2) mittels eines ersten Biegesignals (T1) des ersten Biegesensors (B1) und mittels eines zweiten Biegesignals (T2) des zweiten Biegesensors (B2);
    Auswertemittel (M2) zum Ermitteln eines für die Torsion charakteristischen Biegesignals (CT) durch Addition des ersten Biegesignals (T1) und des zweiten Biegesignals (T2).

11. Vorrichtung (V) nach Anspruch 10,
    **dadurch gekennzeichnet, dass**
    das Messmittel (M1) vor einer Durchführung der Torsion ein erstes Basisbiegesignal (BT1) des ersten Biegesensors (B1) und ein zweites Basisbiegesignal (BT2) des zweiten Biegesensors (B1) misst;

das Auswertemittel (M2) zum Erstellen eines charakteristischen Basisbiegesignals (BCT) durch Addieren des ersten und des zweiten Basisbiegesignals (BT1, BT2) und zum Subtrahieren des charakteristischen Basisbiegesignals (BCT) von dem ermittelten charakteristischen Biegesignal (CT).

**12.** Vorrichtung (V) nach einem der Ansprüche 10 bis 11,
**dadurch gekennzeichnet, dass**
die Biegesensoren (B1, B2) an der Oberfläche des Körpers (K) nahezu parallel zur Achse (A) der Torsion angebracht sind.

**13.** Vorrichtung (V) nach einem der Ansprüche 10 bis 12,
**dadurch gekennzeichnet, dass**
die Biegesensoren (B1, B2) eine jeweilige sensitive Zone (Z1, Z2) aufweisen, wobei die sensitiven Zonen (Z1, Z2) mit einer jeweiligen örtlichen Raumausdehnung in Richtung der Achse (A) angeordnet sind.

**14.** Vorrichtung (V) nach einem der Ansprüche 10 bis 13,
**dadurch gekennzeichnet, dass**
das Auswertemittel (M2) ein zu der Torsion gehörenden Torsionswinkel (TR) mittels des charakteristischen Biegesignals (CT) auf Basis einer Umrechnungsfunktion (UF) erstellt.

**15.** Vorrichtung (V) nach einem der Ansprüche 10 bis 14,
**dadurch gekennzeichnet, dass**
das Messmittel (M1) ein jeweiliges Paar erster und zweiter Biegesignale (T1, T2) über der Zeit (t) misst,
das Auswertemittel (M2) einen Zeitverlauf der Torsion durch Generieren des jeweiligen charakteristischen Biegesignals (CT) jeweiliger Paare des ersten und zweiten Biegesignals (T1, T2) ermittelt.

**16.** Vorrichtung (V) nach einem der Ansprüche 10 bis 15,
**dadurch gekennzeichnet, dass**
der Körper (K) als Rücken eines Patienten und die Achse (A) als Wirbelsäule des Patienten ausgebildet sind.

**17.** Vorrichtung (V) nach einem der Ansprüche 10 bis 16,
**dadurch gekennzeichnet, dass**
Biegesensoren eingesetzt werden, die als faseroptische Biegesensoren ausgebildet sind.

**18.** Verwendung der Vorrichtung (V) nach einem der Ansprüche 10 bis 17,
**dadurch gekennzeichnet, dass**
die Vorrichtung zur Messung der Torsion eines menschlichen Körperteils, insbesondere des Rückens mit der Wirbelsäule als Achse der Torsion, verwendet wird.

**Claims**

**1.** Method for measuring a torsion of a body (K) with the aid of flexion sensors (B1, B2), with the flexion sensors being arranged on a surface of the body (K),
with the following steps:

Measuring a flexion at the flexion sensors (B1, B2) arising from the torsion in each case by means of a first flexion signal (T1) of the first flexion sensor (B1) and by means of a second flexion signal (T2) of the second flexion sensor (B2),
with the flexion of the first and second flexion sensor (B1, B2) in a same spatial direction causing an increase of the first flexion signal (T1) and a decrease of the second flexion signal (T2);
Determining a flexion signal (CT) characteristic for the torsion by addition of the first flexion signal (T1) and the second flexion signal (T2).

**2.** Method according to claim 1,
**characterized in that,**
before the torsion is carried out, a first basic flexion signal (BT1) of the first flexion sensor (B1) and a second basic flexion signal (BT2) of the second flexion sensor (B1) is measured,
a characteristic basic flexion signal (BCT) is created by addition of the first and of the second basic flexion signal (BT1, BT2),
after the torsion has been carried out the characteristic basic flexion signal (BCT) is subtracted from the characteristic flexion signal (CT) determined.

3. Method according to one of the previous claims,
**characterized in that**
flexion sensors (B1, B2) are used which are attached to the surface of the body (K) almost in parallel to axis (A) of the torsion.

4. Method according to one of the previous claims,
**characterized in that**
flexion sensors (B1, B2) are used which have a respective sensitive zone (Z1, Z2) with a respective local spatial extension in the direction of the axis (A).

5. Method according to one of the previous claims,
**characterized in that**
a torsion angle (TR) belonging to the torsion is created by means of the characteristic flexion signal (CT) on the basis of a conversion function (UF).

6. Method according to one of the previous claims,
**characterized in that**
a respective pair of first and second flexion signals (T1, T2) are measured over time (t),
a time curve of the torsion is determined by generation of the respective characteristic flexion signal (CT) of respective pairs of the first and second flexion signal (T1, T2).

7. Method according to one of the previous claims,
**characterized in that**
the body (K) is embodied as a patient's back and the axis (A) as the patient's spinal column.

8. Method according to one of the previous claims,
**characterized in that**
flexion sensors are used that are embodied as fibre optic flexion sensors.

9. Method according to one of the previous claims,
**characterized in that,**
before the determination of the characteristic flexion signal a weighting of the respective flexion signals is undertaken.

10. Device for measuring a torsion of a body (K),
with the following means:

First flexion sensor (B1) and second flexion sensor (B2), which are arranged on a surface of the body (K), with the flexion of the first and second flexion sensor (B1, B2) arising in a same spatial direction causing an increase in the first flexion signal (T1) and a decrease in the second flexion signal (T2);
Measurement means (M1) for measuring the respective flexion at the flexion sensors (B1, B2) arising from the torsion by means of a first flexion signal (T1) of the first flexion sensor (B1) and by means of a second flexion signal (T2) of the second flexion sensor (B2);
Evaluation means (M2) for determining a characteristic flexion signal (CT) for the torsion by addition of the first flexion signal (T1) and the second flexion signal (T2).

11. Device (V) according to claim 10,
**characterized in that,**
before the torsion is carried out, the measurement means (M1) measure a first basic flexion signal (BT1) of the first flexion sensor (B1) and a second basic flexion signal (BT2) of the second flexion sensor (B1);
the evaluation means (M2) create a characteristic basic flexion signal (BCT) for addition of the first and the second basic flexion signal (BT1, BT2) and for subtraction of the characteristic basic flexion signal (BCT) from the characteristic flexion signal (CT) determined.

12. Device (V) according to one of the claims 10 to 11,

**characterized in that**
the flexion sensors (B1, B2) are arranged on the surface of the body (K) almost in parallel to the axis (A) of the torsion.

13. Device (V) according to one of the claims 10 to 12,
    **characterized in that**
    the flexion sensors (B1, B2) each have a sensitive zone (Z1, Z2), with the sensitive zones (Z1, Z2) with a respective local spatial extension being arranged in the direction of the axis (A).

14. Device (V) according to one of the claims 10 to 13,
    **characterized in that**
    evaluation means (M2) create a torsion angle (TR) belonging to the torsion by means of the characteristic flexion signal (CT) based on a conversion function (UF).

15. Device (V) according to one of the claims 10 to 14,
    **characterized in that**
    the measurement means (M1) measure a respective pair of first and second flexion signals (T1, T2) over time (t), the evaluation means (M2) determine a time curve of the torsion by generating the respective characteristic flexion signal (CT) of respective pairs of the first and second flexion signal (T1, T2).

16. Device (V) according to one of the claims 10 to 15,
    **characterized in that**
    the body (K) is embodied as a patient's back and the axis (A) as the patient's spinal column.

17. Device (V) according to one of the claims 10 to 16,
    **characterized in that**
    flexion sensors are used that are embodied as fibre optic flexion sensors.

18. Use of the device (V) according to one of the claims 10 to 17,
    **characterized in that,**
    the device is used for measurement of the torsion of a part of the human body, especially of the back with the spinal column as the axis of the torsion.

**Revendications**

1. Procédé de mesure d'une torsion d'un corps (K) à l'aide de capteurs (B1, B2) de flexion, les capteurs de flexion étant mis sur une surface du corps ( K ),
   comprenant les stades suivantes :

   on mesure une flexion respective survenant par la torsion sur les capteurs (B1, B2) de flexion à l'aide d'un premier signal ( T1 ) de flexion du premier capteur ( B1 ) de flexion et à l'aide d'un deuxième signal ( T2 ) de flexion du deuxième capteur ( B2 ) de flexion, la flexion du premier et du deuxième capteur ( B1, B2 ) de flexion dans une même direction de l'espace provoquant une augmentation du premier signal ( T1 ) de flexion et une diminution du deuxième signal ( T2 ) de flexion ;
   on détermine un signal ( CT ) de flexion caractéristique de la torsion par addition du premier signal ( T1 ) de flexion et du deuxième signal ( T2 ) de flexion.

2. Procédé suivant la revendication 1,
   **caractérisé en ce que**
   avant d'effectuer la torsion, on mesure un premier signal ( BT1 ) de flexion de base du premier capteur ( B1 ) de flexion et un deuxième signal ( BT2 ) de flexion de base du deuxième capteur ( B1 ) de flexion,
   on établit un signal ( BCT ) de flexion de base caractéristique par addition du premier et du deuxième signal ( BT1, BT2 ) de flexion de base,
   après avoir effectué la torsion, on soustrait le signal ( BCT ) de flexion de base caractéristique du signal ( CT ) de flexion caractéristique déterminé.

3. Procédé suivant l'une des revendications précédentes,
   **caractérisé en ce que**

on utilise des capteurs ( B1, B2 ) de flexion, qui sont mis sur la surface du corps ( K ) à peu près parallèlement à l'axe ( A ) de la torsion.

4. Procédé suivant l'une des revendications précédentes,
   **caractérisé en ce que**
   on utilise des capteurs ( B1, B2 ) de flexion, qui ont une zone ( Z1, Z2 ) respective sensible ayant une étendue dans l'espace local respectif dans la direction de l'axe ( A ).

5. Procédé suivant l'une des revendications précédentes,
   **caractérisé en ce que**
   on établit un angle ( TR ) de torsion appartenant à la torsion à l'aide du signal ( CT ) de flexion caractéristique sur la base d'une fonction ( UF ) de conversion.

6. Procédé suivant l'une des revendications précédentes,
   **caractérisé en ce que**
   on mesure une paire respective de premier et de deuxième signaux ( T1, T2 ) de flexion en fonction du temps ( t ), on détermine une courbe en fonction du temps de la torsion en produisant le signal ( CT ) de flexion caractéristique respective de paire respective du premier et du deuxième signal ( T1, T2 ) de flexion.

7. Procédé suivant l'une des revendications précédentes,
   **caractérisé en ce que**
   le corps ( K ) est constitué sous la forme du dos d'un patient et l'axe ( A ) sous la forme d'une colonne vertébrale du patient.

8. Procédé suivant l'une des revendications précédentes,
   **caractérisé en ce que**
   on utilise des capteurs de flexion, qui sont constitués sous la forme de capteurs de flexion à fibre optique.

9. Procédé suivant l'une des revendications précédentes,
   **caractérisé en ce que**
   avant la détermination du signal de flexion caractéristique, on effectue une pondération des signaux de flexion respectifs.

10. Dispositif ( V ) de mesure d'une torsion d'un corps ( K ), comprenant les moyens suivantes :

    un premier capteur ( B1 ) de flexion et un deuxième capteur ( B2 ) de flexion, qui sont mis sur la surface du corps ( K ), la flexion du premier et du deuxième capteur ( B1, B2 ) de flexion dans une même direction de l'espace provoquant une augmentation du premier signal ( T1 ) de flexion et une diminution du deuxième signal ( T2 ) de flexion ;
    des moyens ( M1 ) de mesure pour mesurer la flexion respective survenant par la torsion sur les capteurs ( B1, B2 ) de flexion au moyen d'un premier signal ( T1 ) de flexion du premier capteur ( B1 ) de flexion et au moyen d'un deuxième signal ( T2 ) de flexion du deuxième capteur ( B2 ) de flexion ;
    des moyens ( M2 ) d'exploitation pour déterminer un signal ( CT ) de flexion caractéristique de la torsion par addition du premier signal ( T1 ) de flexion et du deuxième signal ( T2 ) de flexion.

11. Dispositif ( V ) suivant la revendication 10,
    **caractérisé en ce que**
    les moyens ( M1 ) de mesure mesurent avant d'effectuer la torsion un premier signal ( BT1 ) de flexion de base du premier capteur ( B1 ) de flexion et un deuxième signal ( BT2 ) de flexion de base du deuxième capteur ( B1 ) de flexion ;
    les moyens ( M2 ) d'exploitation établissent un signal ( BCT ) de flexion de base caractéristique en additionnant le premier et le deuxième signal ( BT1, BT2 ) de flexion de base et en soustrayant le signal ( BCT ) de flexion de base caractéristique du signal ( CT ) de flexion caractéristique déterminé.

12. Dispositif ( V ) suivant l'une des revendications 10 à 11,
    **caractérisé en ce que**
    les capteurs ( B1, B2 ) de flexion sont mis à la surface du corps ( K ) à peu près parallèlement à l'axe ( A ) de la torsion.

**13.** Dispositif ( V ) suivant l'une des revendications 10 à 12,
**caractérisé en ce que**
les capteurs ( B1, B2 ) de flexion ont une zone ( Z1, Z2 ) sensible respective, les zones ( Z1, Z2 ) sensibles étant disposées en ayant une étendue dans l'espace locale respective dans la direction de l'axe ( A ).

**14.** Dispositif ( V ) suivant l'une des revendications 10 à 13,
**caractérisé en ce que**
les moyens ( M2 ) d'exploitation établissent un angle ( TR ) de torsion pour la torsion au moyen du signal ( CT ) de flexion caractéristique sur la base d'une fonction ( UF ) de conversion.

**15.** Dispositif ( V ) suivant l'une des revendications 10 à 14,
**caractérisé en ce que**
les moyens ( M1 ) de mesure mesurent une paire respective de premier et deuxième signaux ( T1, T2 ) de flexion en fonction du temps ( t ),
les moyens ( M2 ) d'exploitation déterminent une courbe dans le temps de la torsion en produisant le signal ( CT ) de flexion respective de paire respective du premier et du deuxième signal ( T1, T2 ) de flexion.

**16.** Dispositif ( V ) suivant l'une des revendications 10 à 15,
**caractérisé en ce que**
le corps ( K ) est constitué sous la forme du dos d'un patient et l'axe ( A ) sous la forme de la colonne vertébrale du patient.

**17.** Dispositif ( V ) suivant l'une des revendications 10 à 16,
**caractérisé en ce que**
sont utilisés des capteurs de flexion, qui sont constitués en capteurs de flexion à fibre optique.

**18.** Utilisation du dispositif ( V ) suivant l'une des revendications 10 à 17,
**caractérisée en ce que**
on utilise le dispositif pour mesurer la torsion d'une partie du corps humain, notamment du dos, en ayant la colonne vertébrale comme axe de la torsion.

FIG 1A   Stand der Technik

FIG 1B   Stand der Technik

FIG 1C   Stand der Technik

FIG 2

FIG 3

## FIG 4

## FIG 5

## FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0035345 A
- US 5097252 A
- US 6127672 A